Europäisches Patentamt

European Patent Office

Office européen des brevets

(11) Publication number: **0 211 576**
**B1**

# EUROPEAN PATENT SPECIFICATION

(45) Date of publication of the patent specification:
24.10.90

(51) Int. Cl.⁵: **A61F 2/24**

(21) Application number: **86305710.5**

(22) Date of filing: **24.07.86**

(54) **A heart valve prosthesis.**

(30) Priority: **24.07.85 US 758611**

(43) Date of publication of application:
**25.02.87 Bulletin 87/9**

(45) Publication of the grant of the patent:
**24.10.90 Bulletin 90/43**

(84) Designated Contracting States:
**AT BE CH DE FR GB IT LI LU NL SE**

(56) References cited:
**EP-A- 0 079 844**
**EP-A- 0 133 608**
**DE-B- 2 815 756**

(73) Proprietor: **McQueen, David M., Courant Institute of
Mathematical Sciences 251 Mercer Street, New York
New York 10012(US)**
Proprietor: **Peskin, Charles S., Courant Institute of
Mathematical Sciences 251 Mercer Street, New York
New York 10012(US)**

(72) Inventor: **McQueen, David M., Courant Institute of
Mathematical Sciences 251 Mercer Street, New York
New York 10012(US)**
Inventor: **Peskin, Charles S., Courant Institute of
Mathematical Sciences 251 Mercer Street, New York
New York 10012(US)**

(74) Representative: **Lucas, Brian Ronald, Lucas, George &
Co. 135 Westhall Road, Warlingham Surrey CR3 9HJ(GB)**

## Description

This invention relates to a heart valve prosthesis.

Various types of heart valve prosthesis have been developed which operate hemodynamically as a result of the pumping action of the heart in which they generally function as check valves.

One type of heart valve prosthesis commercially available is the St. Jude valve, which comprises a valve body having a passageway for the flow of blood therethrough from upstream to downstream, and first and second leaflets which are pivotally supported in the passageway and movable between a closed position inhibiting blood flow through said passageway and an open position allowing blood flow therethrough. The first and second leaflets are flat.

Another type of heart valve prosthesis is the Hemex valve which is a bileaflet valve in which the leaflets are curved in a plane parallel to the pivot axis of the leaflets.

Figure 12 of EP-A-0 079 844 discloses a heart valve prosthesis comprising, a valve body having a passageway for the flow of blood therethrough from upstream to downstream, and first and second leaflets individually pivotally supported in said passageway and pivotal between a closed position inhibiting blood flow through said passageway and an open position allowing blood flow therethrough, wherein said first and second leaflets are curved in a plane normal to their respective pivot axis and are arranged so that the convex sides of said first and second leaflets face each other when said leaflets are in said open position.

As can be derived from Figures 15 and 16 of EP-A-0 079 844, when the leaflets are open the major flow of blood will be between the leaflets with relatively stagnant areas to either side of the leaflets.

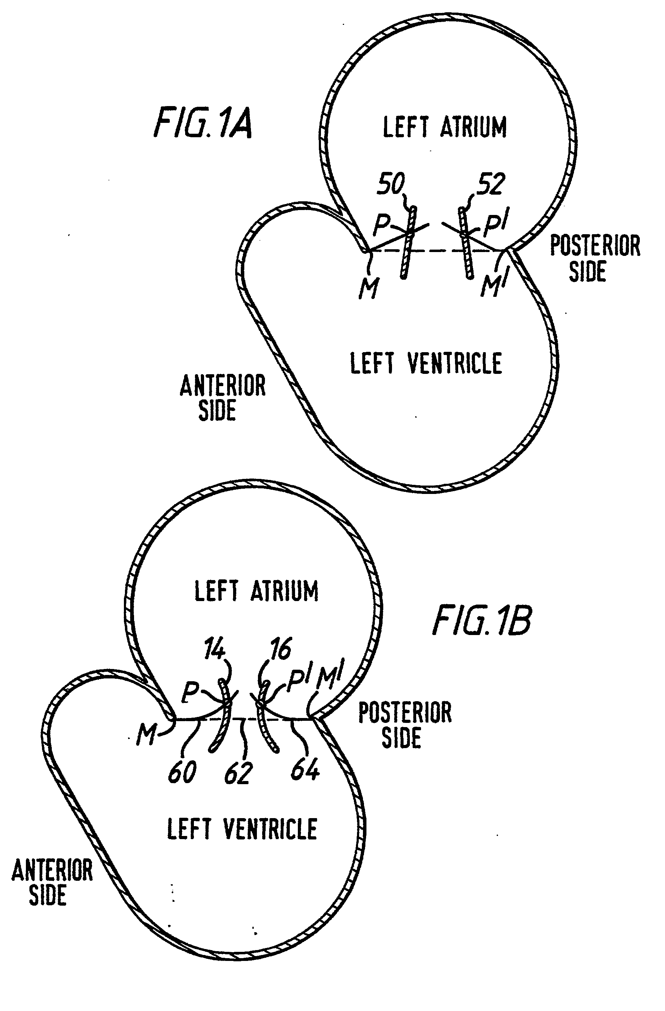

Figure 1A hereto shows an arrangement in which flat leaflets are pivoted so that when the flat leaflets are open three channels are formed, viz: a central channel, bounded by two side channels.

Whilst the arrangement is preferable to that disclosed in Figure 12 of EP-A-0 079 844 the maximum flow through the central channel is low compared with that in the two side channels.

The present invention is characterized in that the first and second leaflets have concave sides which face upstream when in the closed position and are pivotally supported greater than one half the width of the leaflets from the outer edge of said leaflets.

Advantageously, said first and second leaflets are symmetrical.

Preferably, said pivot axes lie in a common plane which is perpendicular to the direction of flow of blood through said heart valve prosthesis in use.

Advantageously, the first and second leaflets are pivotally supported approximately two thirds of the width of the leaflets from the outer edges of the leaflets.

Preferably, the curvature of said leaflets is circular.

Advantageously, the curvature C of said leaflets is in the range of approximately .4 to .7 wherein $C = d_0/r_0$, and

r = the radius of curvature of the leaflets, and

$d_0$ = the length of the chord of said leaflets.

Preferably, the heart valve prosthesis includes means in said valve body for limiting the maximum angle of opening of said leaflets to approximately ninety degrees from a plane transverse to said passageway.

Advantageously, the maximum angle of opening is in the range of approximately eighty-five to ninety degrees.

For a better understanding of the present invention and to show how the same may be carried into effect, reference will now be made by way of example, to the accompanying drawings in which:

Figure 1A is a schematic of a prior art heart valve prosthesis with flat leaflets in the mitral position shown in both the open and closed positions;

Figure 1B is a view similar to Figure 1A but showing a heart valve prosthesis in accordance with the present invention;

Figures 2A, 2B and 2C are schematic views of the heart valve prosthesis shown in Figure 1B illustrating various parameters;

Figure 3 is a graph illustrating the opening angles of the anterior and posterior leaflets of the conventional heart valve prosthesis shown in Figure 1A as a function of time;

Figure 4A is a graph illustrating the peak velocity in each of the three openings of the conventional heart valve prosthesis shown in Figure 1A as a function of opening angle;

Figure 4B is a graph similar to that of 4A but with the heart valve prosthesis shown in Figure 1B;

Figure 5 is a graph illustrating the peak velocity in each of the three openings of the heart valve prosthesis shown in Figure 1B as a function of the axis pivot point location with a maximum valve opening of 85 degrees;

Figure 6A is a graph of the minimum peak velocity as a function of the pivot point location for various leaflet curvatures with a maximum valve opening of 85 degrees;

Figure 6B is a graph similar to Figure 6A for a maximum valve opening of 90 degrees;

Figures 7A and 7B are enlarges views of parts of Figure 6A and 6B, respectively;

Figure 8A is a graph of maximum over pivot point location of minimum peak velocity as a function of leaflet curvature with a maximum valve opening of 85 degrees;

Figure 8B is a graph similar to Figure 8A but with a valve maximum opening of 90 degrees;

Figure 9A is a graph of stroke volume as a function of pivot point location for various leaflet curvatures with a maximum valve opening of 85 degrees;

Figure 9B is a graph similar to that of Figure 9A for a maximum valve opening of 90 degrees;

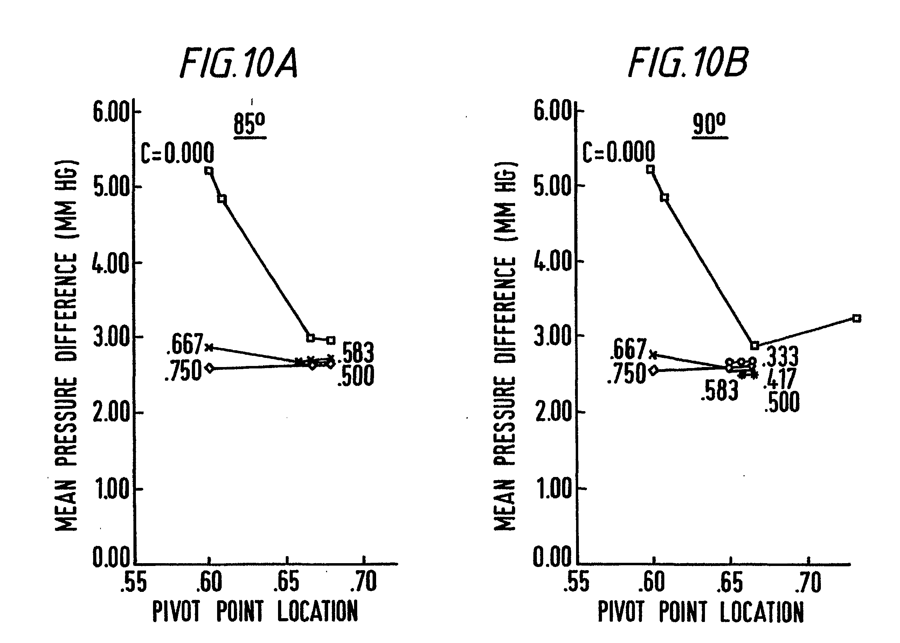

Figure 10A is a graph of mean transvalvular pressure difference as a function of pivot point location for various leaflet curvatures with a maximum valve opening of 85 degrees;

Figure 10B is a graph similar to that of Figure 10A but with a maximum valve opening of 90 degrees;

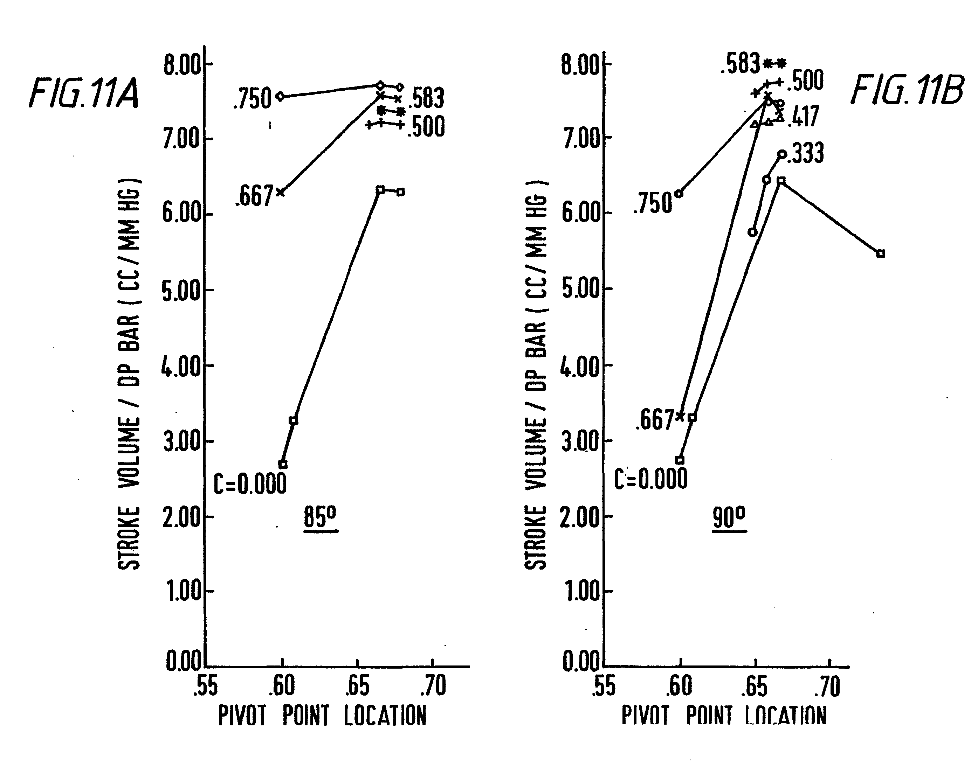

Figure 11A is a graph of the net stroke volume/mean forward pressure difference or hemodynamic benefit/cost ratio as a function of pivot point location for various leaflet curvatures with a maximum valve opening of 85 degrees;

Figure 11B is a graph similar to that of Figure 11A but with a maximum valve opening of 90 degrees;

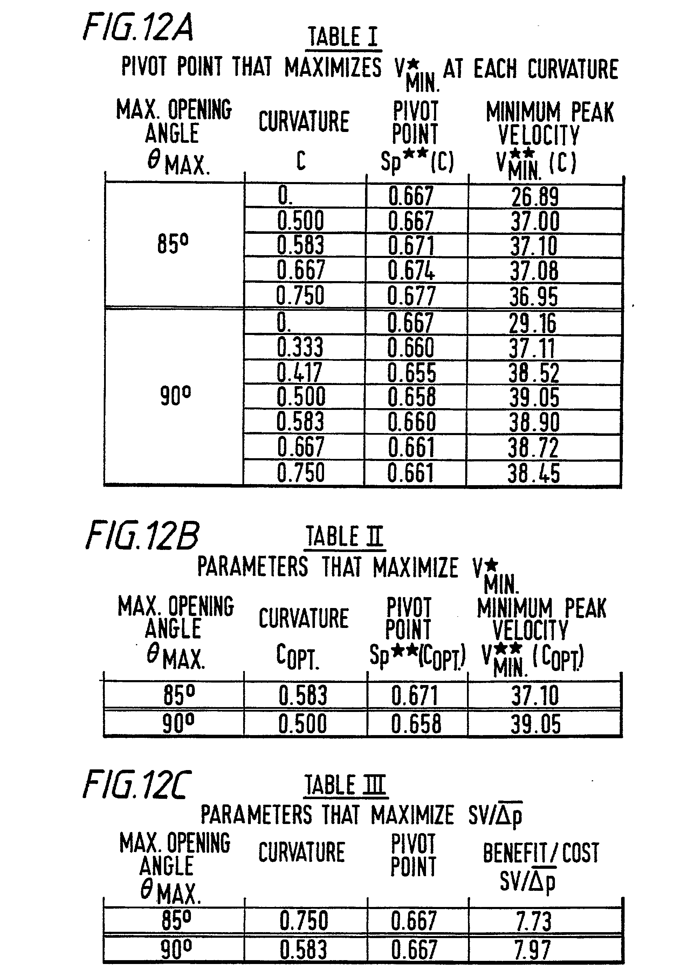

Figure 12A, 12B and 12C are respectively tables I, II and III of various data;

Figure 13 is an elevational view, in cross-section, of a preferred embodiment of a heart valve prosthesis in accordance with the present invention; and

Figure 14 is a top plan view of the embodiment shown in Figure 13.

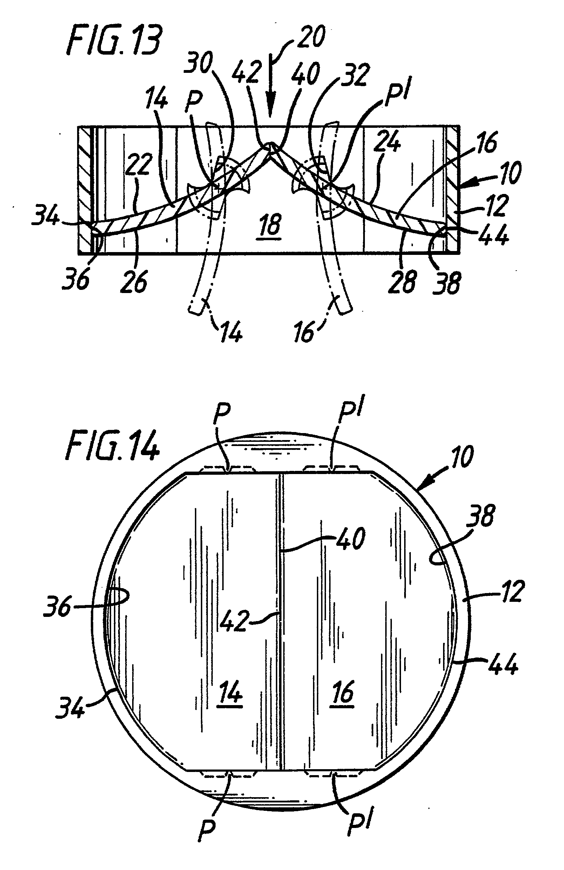

Referring now to the drawings, and particularly to Figures 13 and 14, there is shown a heart valve prosthesis which is generally identified by reference numeral 10. The heart valve prosthesis 10 includes an annular body 12 which supports first and second leaflets 14 and 16 which pivot about axes P, P' respectively to control the flow of blood through a central passageway 18. The normal flow of blood through the valve 10 is in the direction of the arrow 20. Any suitable means may be connected to the annular body 12 for attaching a suturing ring to the annular body 12 to facilitate sewing the valve 10 to the heart tissue as is conventional.

The valve body 12 and leaflets 14 and 16 may be made of any suitable material that is biocompatible and non-thrombogenic, for example, isotropic graphite which is suitably coated with pyrolitic carbon.

The leaflets 14 and 16 are each pivotally supported on respective eccentric positioned axis P and P', The leaflets 14 and 16 are curved in a plane normal to their respective pivot axis. The leaflets 14 and 16 have a concave side 22 and 24, respectively, and a convex side 26 and 28, respectively. The convex sides 26 and 28 face each other when the leaflets 14 and 16 are in the open position and the concave sides 22 and 24 face upstream when the valve is in the closed position. Preferably, the curve of the leaflets 14 and 16 is circular and preferably the leaflets 14 and 16 are flat in a plane parallel to the eccentric axis although the leaflets 14 and 16 could also be curved in a plane parallel to the pivot axis.

The body 12 may include stop shoulders 30 and 32 positioned in the path of movement of the leaflets 14 and 16, respectively, for limiting the maximum opening angle of the leaflets 14 and 16 as will be more fully discussed hereinafter. The leaflets 14 and 16 are generally circular-arc in cross section and are adapted to engage and seat on a valve seat 34,44 in the body 12 when in the closed position by the outside edges 36 and 38 of the leaflets 14 and 16, respectively. The inside edges 40 and 42 are positioned to engage each other when the valve is in the closed position. Blood flowing in the direction of the arrow 20 from upstream to downstream will open the leaflets 14 and 16 until they reach their shoulder stops 30 and 32, respectively. When the downstream flow of blood is discontinued, the back pressure which is present will cause the leaflets 14 and 16 to swing or pivot around the pivot axes P and P', respectively, towards the closed position.

As in the prior art valves using two flat leaflets, the present valve 10 when opened has three separate channels for forward blood flow, that is, two lateral channels and one central channel. However, both physical measurements and computer studies show that the blood flow in the central channel of the prior art flat leaflets tends to be stagnant relative to the flow in the lateral channels of the valve. Stagnant flow is dangerous because it may lead to valve thrombosis. One of the features of the present invention is the reduction of this problem by curving the valve leaflets 14 and 16 as described above to form a central venturi channel which helps open the leaflets 14 and 16 and also funnels flow into the previously stagnant central channel of the valve.

In addition, various parameters have been considered to improve the performance of the valve 10 to select an optimal valve that not only maximizes the minimum peak velocity in any of the flow channels, but also maximizes the net stroke volume of blood crossing the valve per heart beat and minimizes the mean pressure difference during forward flow, thereby improving the hemodynamic performance. In order to provide an optimal valve, the parameters considered are the position of the pivot points P and P', the radius of curvature of the leaflets 14 and 16, and the maximum opening angle of the leaflets 14 and 16. A computer was used to define the parameters of a model valve and the performance criteria that are used to select an optimal valve.

Referring now to Figs. 1A and 1B, a prior art heart valve prosthesis shown in Fig. 1A having flat leaflets 50 and 52 which are positioned in the mitral annulus between the left ventricle and the left atrium while a valve of the present invention using curved leaflets 14 and 16 is shown in Fig. 1B. The open position of the leaflets 50, 52 and 14 and 16 are shown in heavy dotted lines. The initial (closed) position is indicated by the light solid lines. Points M and M' are the extreme anterior and posterior points of the mitral annulus. Points P and P' are the pivot points. As previously indicated, the prior art valve of Fig. 1A in tests showed that the blood flow in the central channel between the leaflets 50 and 52 tends to be stagnant relative to the flow in the outside channels which may lead to valve thrombosis. On the other hand, the present design of Fig. 1B provides a venturi channel between the leaflets 14 and 16 which not only helps to open the leaflets 14 and 16 but to funnel additional flow into the channel between the leaflets 14 and 16.

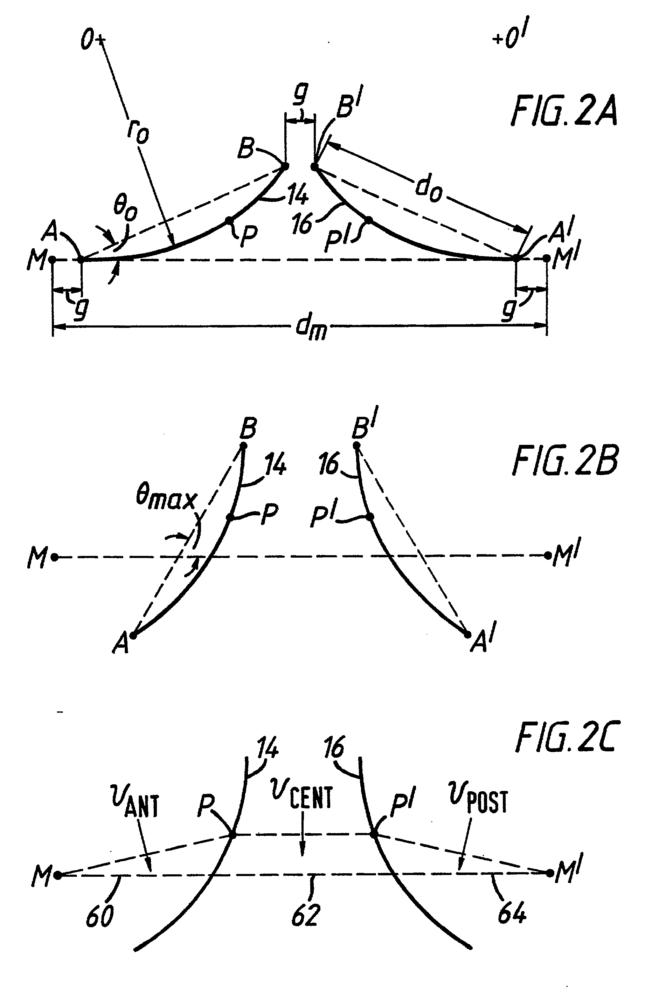

Referring now to Figs. 2A, 2B and 2C, the geometrical construction of a model valve of the present invention is shown in which the valve is shown in its closed configuration in Fig. 2A and in its open configuration in Fig. 2B and Fig. 2C. The valve is treated as symmetrical despite the asymmetry that is inherent in the mitral position. The valve is constructed starting from the points M and M' that define the mitral ring and the diameter of that ring is $d_m$.

The gap distance g separates the ends of the leaflets I4 and I6 from the mitral ring and from each other. Because all boundaries have an effective thickness (of about 2 mesh widths of the computational mesh used in the computer model), these gaps are effectively closed when the valve is in the closed position. The chords AB and A'B' of the leaflets I4 and I6 are constructed in the closed position at an angle $\theta_0$ to the line MM' that defines the level of the mitral ring. Throughout, we take $\theta_0 = 25°$. This 25 degrees initial angle of the leaflets I4 and I6 relative to the mitral ring was chosen to approximately minimize the volume swept out by the leaflets during their opening or closure movement. This result is independent of the position of the pivot points P and P' and the curvature of the leaflets I4 and I6 and roughly minimizes the volume swept for leaflets having a maximum opening angle in the range between 85 degrees and 90 degrees, which opening angle will be further discussed herein.

The leaflets I4 and I6 themselves are the circular arcs APB and A'P'B' with centers of curvature at 0 and 0', respectively, on the upstream (atrial) side of the valve. Let

$r_0$ = radius of curvature of the leaflets
$d_0$ = length of AB = chord of the leaflets
$R = r_0/d_0$
$C = 1/R$

Then R is the dimensionless radius of curvature, and C, the reciprocal of R, is the dimensionless curvature. The special case of a flat valve is characterized by $r_0 = \infty$, $R = \infty$ and $C = 0$. In the flat case the leaflet coincides with the chord AB. We shall use the parameter C to characterize the curvature of the valve. This is one of the principal design parameters.

Another important parameter is the position of the pivot points such as P (which remains fixed relative to the points M and M' as the leaflets pivot). Its location is defined by measuring arc length from the outer border of the leaflet. Let

$s_p$ = length of the arc AP
$s_0$ = length of arc APB
$S_P = s_p/s_0$

Then SP gives the position of the pivot point in dimensionless form. By definition, $0 < S_P < 1$, with the larger values of $S_P$ corresponding to pivot points which are closer to the center of the valve apparatus. In practice, $S_P$ must be chosen, as will be discussed hereinafter, in the neighborhood of 2/3 to achieve reasonable performance. If $S_P < 0.5$, the valve does not open at all.

The final design parameter is the maximum angle of opening of the valve. This angle, $\theta_{max}$, is the angle of the chords AB and A'B' to the plane of the mitral ring, as shown in Fig. 2B. Most of the computations in the current study were conducted with $\theta_{max} = 85°$ or 90°, as will be more fully discussed hereinafter.

Two other of the performance criteria are the net stroke volume (SV) and the mean forward pressure difference ($\overline{\Delta p}$). The net stroke volume is defined as the net volume of blood crossing the plane of the mitral annulus during the time interval when the valve is open. It is the difference between the forward volume and the regurgitant volume associated with the closure movement of the leaflets 14 and 16.

The mean forward pressure difference is defined by the formula

$$\overline{\Delta p} = \frac{1}{T} \int_0^T (p_{LA}(t) - p_{LV}(t))dt$$

where $P_{LA}(t)$ and $P_{LV}(t)$ are the left atrial and left ventricular pressures at time t. The time $t = 0$ is defined as the onset of mitral flow, amd $t = T$ is defined as the time of the first zero-crossing of the mitral flow, which occurs during early ventricular systole. The rationale for this choice of times is that it eliminates an inertial component of the pressure difference. These two performance criteria are combined into a single benefit/cost ratio: $SV/\overline{\Delta p}$. This seems appropriate because SV and $\overline{\Delta p}$ are not really independent measures of performance.

The following performance criterion is concerned with valve thrombosis. Let $Q_{ant}(t)$ be the volume rate of flow crossing the line MP in Fig. 2C, let $Q_{cent}(t)$ be the volume rate of flow crossing the line PP' and let $Q_{post}(t)$ be the volume rate of flow crossing P'M'. Thus $Q_{and}$, $Q_{cent}$, $Q_{post}$ are the anterior 60, central 62 and posterior 64 flows, respectively. Let $A_{ant}$, $A_{cent}$, and $A_{post}$ be the cross-sectional areas of the three openings of the valve. That is, let

$A_{ant}$ = (length of MP) $\cdot$ $L_O$
$A_{cent}$ = (length of PP') $\cdot$ $L_O$
$A_{post}$ = (length of P'M') $\cdot$ $L_O$

where $L_O = 1.84$ cm is a reference length ($L_O$ is approximately the diameter of the mitral ring) that is used to convert length in the two-dimensional model to cross-sectional area.

Next define

$v_{ant}(t) = Q_{ant}(t)/A_{ant}$
$v_{cent}(t) = Q_{cent}(t)/A_{cent}$
$v_{post}(t) = Q_{post}(t)/A_{post}$

The quantities $v_{ant}(t)$, $v_{cent}(t)$, $v_{post}(t)$ have units of velocity; they are the spatially averaged velocity at time t in each of the three openings of the valve. (These velocities are independent of $L_O$ because both Q and A contain factors of $L_O$ in their definition.)

The maximum over time of the velocities $v_{ant}$, $v_{cent}$, and $v_{post}$ will be designated by the symbol *. Thus

$v^*_{nt} = M^*x \{v_{ant}(t)\}$
$v^*_{ent} = M^*x \{v_{cent}(t)\}$
$v^*_{ost} = M^*x \{v_{post}(t)\}$

These peak velocities are important because a low peak velocity implies stagnation and hence a tendency to thrombosis. To avoid thrombosis altogether, we adopt a worst-case criterion. Let

$v^*_{in} = Min \{v^*_{nt}, v^*_{ent}, v^*_{ost}\}$

If $v^*_{in}$ = 30 cm/sec, for example, then a velocity of at least 30 cm/sec is achieved at some time during filling in each opening of the valve. We seek design parameters that make $v^*_{in}$ as large as possible.

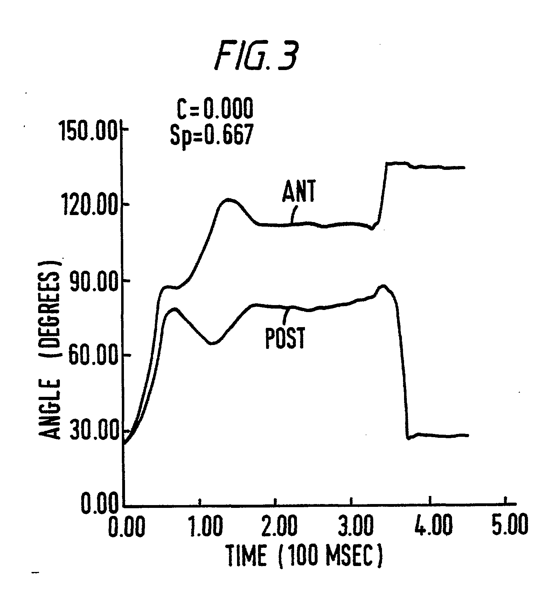

In the case of single-disc valves, good performance can be achieved without any built-in constraint on the opening angle. For the curved leaflet valve, however, we were unable to find any combination of pivot point and curvature for which the valve would exhibit good performance without a constraint. The difficulty is related to asymmetrical movement of the leaflets I4 and I6 which, in turn, must be caused by the asymmetry of the mitral position, since the valve is symmetrical. Fig. 3 shows the opening angle as a function of time for a prior art flat, bileaflet valve effectively unconstrained ($\theta_{max}$ = I35°) pivoted at $S_P$ = 0.67. Note that the anterior leaflet opens much faster than the posterior leaflet and that it becomes caught in the open position. This makes the valve incompetent. Competent performance can be achieved by reducing $S_P$, but then the posterior leaflet fails to open sufficiently and the valve is very stenotic. One way out of this dilemma would be to construct an asymmetrical valve in which the asymmetry of the valve compensates for the asymmetry of the mitral position. In the present study, which is restricted to symmetrical valves, the only solution is to impose a constraint on the maximum opening angle. With such a constraint, and with the pivot point and curvature in an appropriate range both leaflets I4 and I6 hit the constraint (albeit at slightly different times) and the open configuration of the valve is symmetrical.

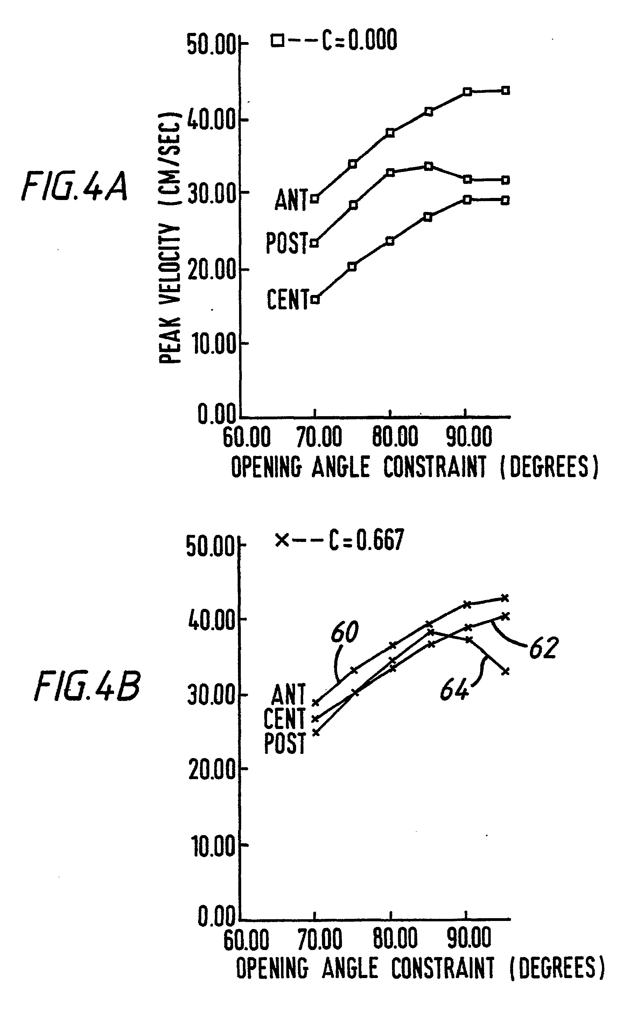

Valve performance has not been systematically optimized with respect to maximum opening angle $\theta_{max}$. Instead we have chosen two reasonable values of $\theta_{max}$ and conducted the rest of the study with these values fixed. The motivation for this choice is shown in Fig. 4, in which peak velocity is plotted in each of the three openings 60, 62, 64 of the valve as a function of the opening angle constraint. The results are shown both for the flat valve of Fig. IA (Fig. 4A) and for a curved valve of the present invention of Fig. IB (Fig. 4B). The pivot point in both cases is $S_P$ = 0.67.

The goal is to make the minimum of the three peak velocities as large as possible. For the flat valve (Fig. 4A) at this pivot point, the minimum peak velocity always occurs in the central opening of the valve. (This has also been observed in vitro.) This minimum peak velocity builds up to its largest value at about a 90 degree opening angle constraint and then levels off. (Competent performance cannot be maintained much beyond 90 degrees, since the anterior leaflet tends to get stuck in the open position and fail to close.) For the curved valve of the present invention, Fig. 4B, the situation is somewhat more complicated, since the smallest peak velocity occurs first in the posterior opening 64, then in the central opening 62, and finally again in the posterior opening 64 as the maximum opening angle increases from 70 degrees to 90 degrees. Here the minimum of the three peak velocities is largest where the center and posterior velocities cross near a maximum opening angle of 85 degrees. These results suggest that opening angle constraints in the range 85 degrees to 90 degrees are most likely to produce good performance.

For the rest of this study, we fix the maximum opening angle at $\theta_{max}$ = 85° or $\theta_{max}$ = 90°. The figures which follow will show results for these two values of $\theta_{max}$ side by side.

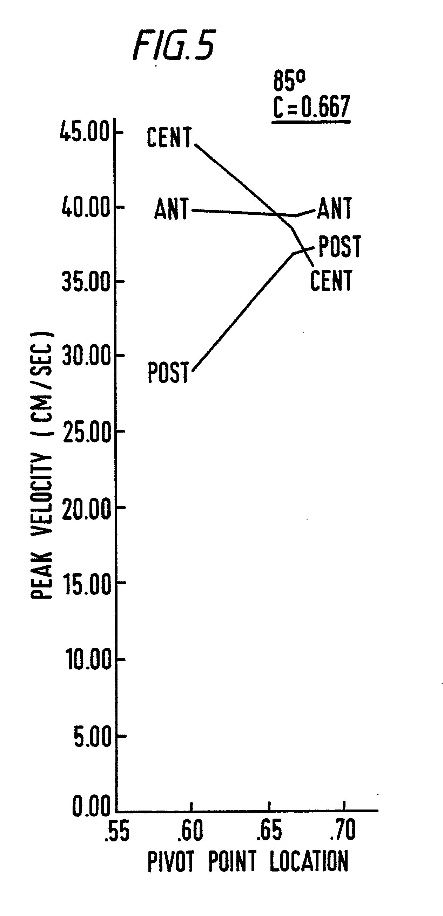

Minimum peak velocity ($v^*_{min}$) varies as a function of pivot point ($S_P$) and curvature (C). Fig. 5 shows a typical plot of peak velocity in the three openings, 60, 62, and 64, of the valve as a function of pivot point with curvature held fixed (in this case C = .667). Note that the different peak-velocity curves intersect and that the minimum peak velocity (heavy line) is largest at the pivot point where the posterior and central peak velocities are equal. In subsequent figures, we plot only the minimum peak velocity without specifying the opening of the valve in which it occurs.

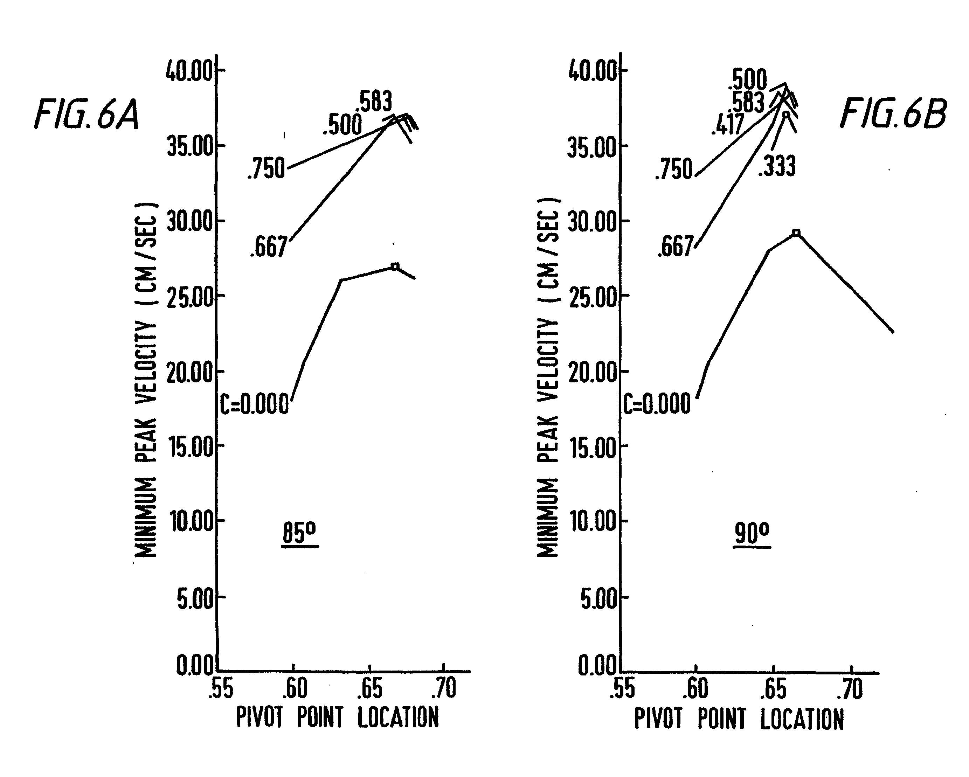

Figs. 6A and 6B plot the minimum peak velocity ($v^*_{min}$) as a function of pivot point ($S_P$) at various curvatures (C = 0, .333, .4I7, .500, .583, .667, .750). There are two remarkable points. First, at every curvature there is a pivot point that maximizes the minimum peak velocity. These pivot points are clustered around $S_P$ = 0.67. Second, the curved valves have substantially higher values of $v^*_{min}$ (minimum peak velocity) than the flat valves of Fig. IA. In fact, by curving the leaflets I4 and I6, as shown in Fig. IB, one can increase $v^*_{in}$ by about I0 cm/sec (more than 30%) in comparison with the best flat valve.

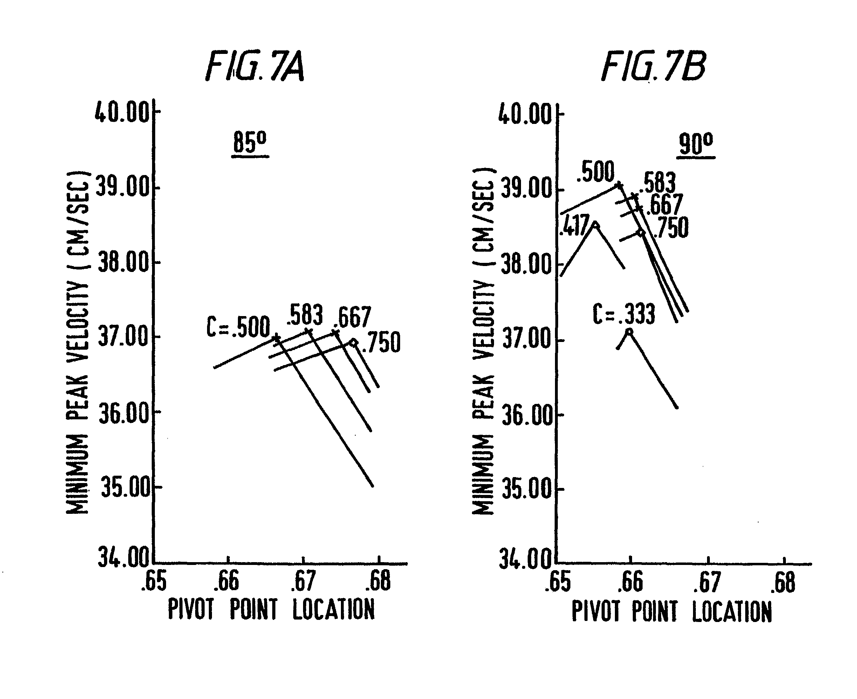

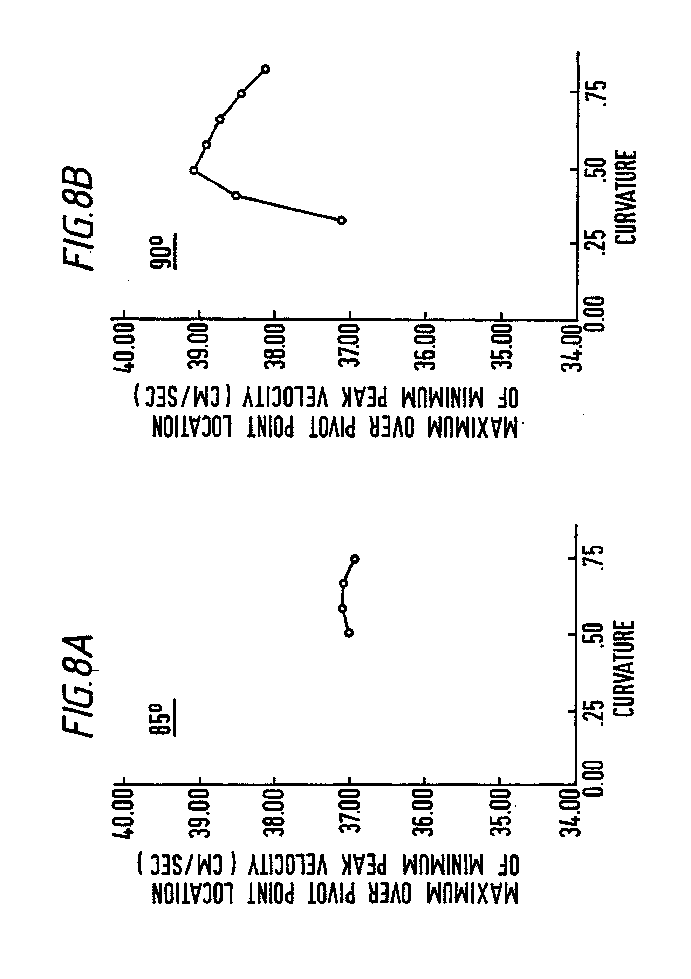

The optimal combination of pivot point and curvature can be found by expanding Figs. 6A and 6B in the neighborhood of the best valves. The result is shown in Figs. 7A and 7B. For each curvature there is an optimal pivot point, denoted by $S^{**}_P$ (C) which maximizes $v^*_{in}$. The value of $v^*_{min}$ at this optimal pivot point is denoted by $v^{**}_{min}$ (C). Figs. 8A and 8B are plots of $v^{**}_n$ as a function of C.

The values of $v^{**}_{min}$ and $S^{**}_P$ are listed in Table I (Figs. I2A). For each value of $\theta_{max}$ there is one curvature, denoted by $C_{opt}$, which results in the largest value of $v^{**}_{min}$. The values of $C_{opt}$, $S^{**}_P$ ($C_{opt}$) and $v^{**}_{min}$ ($C_{opt}$) for $\theta_{max}$ = 85° and 90° are listed in Table II (Fig. I2B). These values define the two best valves in the study.

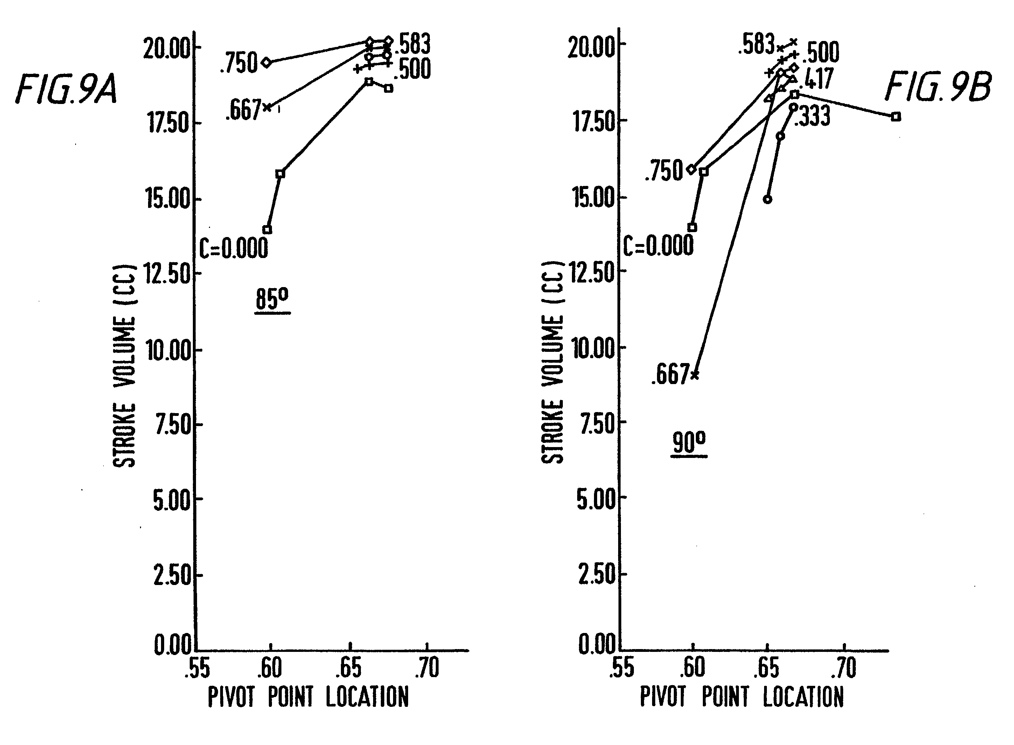

Hemodynamic performance is another important criteria. The foregoing choice of an optimal valve was based on considerations relating to stagnation and thrombosis. Considering the design problem from a hemodynamic (pressure-flow) viewpoint, Figs. 9A and 9B plot the stroke volume (i.e., the net volume of blood crossing the mitral valve during one heart-beat) as a function of pivot point at several curvatures. Figs. I0A and I0B plot the mean pressure difference across the valve in the same way. Figs. IIA and IIB combine these two performance criteria into a single benefit/cost ratio: the quotient of the net stroke volume and the mean pressure difference across the valve.

At each curvature in Figs. IIA and IIB there is an optimal pivot point. As before these optimal pivot points are clustered in the neighborhood of $S_P$ = .667. Starting from the flat valve, C = 0.000, and increasing the curvature, we find that performance (the quotient of stroke volume and mean pressure difference) first improves dramatically and then falls off. Of the valves that we have tested, those with the best hemodynamic performance are listed in Table III (Fig. l2C).

In choosing the best overall valve, how much weight should be given to the minimum peak velocity (a criterion related to thrombosis) in comparison with the hemodynamic benefit/cost ratio of the net stroke volume divided by the mean pressure difference? Fortunately, there is no real need to answer this question because the two criteria lead to very similar conclusions (compare Table II and Table III). Moreover, we can adopt either criterion without much reduction in performance as measured by the other criterion. Suppose, for example, that we select the best valve by the criterion of maximizing $v^*_{min}$ (the minimum peak velocity). While this valve is suboptimal with respect to hemodynamic performance, the reduction in $SV/\Delta p$ (the ratio of stroke volume to mean pressure difference) is only 3.8% for the valve with $\theta_{max}$ = 85° and only 3.5% for the valve with $\theta_{max}$ = 90°. We are therefore in the fortunate situation of being able to select the valve which will minimize thrombosis while sacrificing very little in terms of hemodynamic performance.

In conclusion, a built-in contraint on the maximum angle of opening ($\theta_{max}$) is a necessary design feature of symmetrical butterfly bileaflet valves for the mitral position. The parameter $\theta_{max}$ should be set in the approximate range of 85 degrees - 90 degrees.

With $\theta_{max}$ = 85°, the valve with least stagnation (technically, the valve with the largest value of $v^*_{min}$, the minimum peak velocity) has pivot point $S_P$ = 0.67l and curvature C = 0.583. With $\theta_{max}$ = 90°, the valve with least stagnation has $S_P$ = 0.658 and C = 0.500.

These valves are also nearly optimal from a hemodynamic viewpoint. That is, they come close (within 4%) to achieving the optimal value of net stroke volume divided by mean forward pressure difference.

The most important conclusion is that modest curvature of the leaflets l4 and l6 can make a substantial improvement in the performance of butterfly bileaflet valves. The type of curvature used is in a plane perpendicular to the pivot axes P and P' of the valve l0 and in a direction such that the open valve has a slight waist, like reversed parentheses: )(. When the optimal curvature is selected, the best valve of this type is superior to the best flat valve both with regard to the minimum peak velocity (which should be larger to prevent stagnation and thrombosis) and also with regard to the ratio of net stroke volume to mean forward pressure difference. For the two constraint angles that are considered (85° and 90°) the improvements in minimum peak velocity are 38% and 34%, while the improvements in the hemodynamic benefit/cost ratio are l6% and 20%.

These conclusions are predictions made with the help of a computer model to produce the charts and tables of Figs 3-l2.

## Claims

1. A heart valve prosthesis (10) comprising a valve body (12) having a passageway (18) for the flow of blood therethrough from upstream to downstream, and first and second leaflets (14, 16) individually pivotally supported in said passageway (18) and pivotal between a closed position inhibiting blood flow through said passageway (18) and an open position allowing blood flow therethrough, wherein said first and second leaflets (14, 16) are curved in a plane normal to their respective pivot axis and are arranged so that the convex sides (26, 28) of said first and second leaflets (14, 16) face each other when said leaflets (14, 16) are in said open position, characterized in that the concave (22, 24) sides of said first and second leaflets (14, 16) face upstream when in the closed position and are pivotally supported (P, P') greater than one half the width of the leaflet (14, 16) from the outer edges (36, 38) of said leaflets (14, 16).

2. A heart valve prosthesis as claimed in Claim 1, characterized in that said first and second leaflets (14, 16) are symmetrical.

3. A heart valve prosthesis as claimed in Claim 1 or 2, characterized in that said pivot axes lie in a common plane which is perpendicular to the direction of flow of blood through said heart valve prosthesis in use.

4. A heart valve prosthesis as claimed in Claim 1, 2 or 3, characterized in that said first and second leaflets (14, 16) are pivotally supported (P, P') approximately two thirds of the width of the leaflets (14, 16) from the outer edges (36, 38) of said leaflets.

5. A heart valve prosthesis as claimed in Claim 1, 2, 3 or 4, characterized in that the curvature of said leaflets (14, 16) is circular.

6. A heart valve prosthesis as claimed in Claim 5, characterized in that the curvature C of said leaflets is in the range of approximately .4 to .7 wherein $C = d_0/r_0$, and $r_0$ the radius of curvature of the leaflets, and $d_0$ = the length of the chord of said leaflets.

7. A heart valve prosthesis as claimed in any preceding Claim, characterized in that it includes means (30, 32) in said valve body for limiting the maximum angle of opening of said leaflets to approximately ninety degrees from a plane (M-M') transverse to said passageway.

8. A heart valve prosthesis as claimed in Claim 7, characterized in that the maximum angle of opening is in the range of approximately eighty-five to ninety degrees.

## Patentansprüche

1. Herzklappenprothese (10) die einen Klappenkörper (12) mit einem Durchgang (18) für die Blutströmung von oben nach unten durch diese hindurch und ein erstes und zweites Blättchen (14, 16) umfaßt, die einzeln in diesem Durchgang (18) drehbar gehalten werden und zwischen einer geschlossenen Position, die die Blutströmung durch den Durchgang (18) hemmt und einer offenen Position drehbar sind, die die Blutströmung durch diese hindurch gestattet, wobei das erste und zweite Blättchen (14, 16) in einer zu ihren entsprechenden Drehachsen normalen Ebene gekrümmt sind und so angeordnet sind, daß die konvexen Seiten (26, 28) des ersten und zweiten Blättchens (14, 16) einander gegenüberstehen, wenn die Blättchen (14, 16) in offener Position sind, dadurch gekennzeichnet, daß die konkaven (22, 24) Seiten des ersten und zweiten Blättchens (14, 16) stromaufwärts zeigen, wenn sie in der geschlossenen Position sind und bei mehr als der Hälfte der Breite des Blättchens (14, 16) von den Außenkanten (36, 38) der Blättchen (14, 16) drehbar gehalten werden (P, P').

2. Herzklappenprothese nach Anspruch 1, dadurch gekennzeichnet, daß das erste und das zweite Blättchen (14, 16) symmetrisch sind.

3. Herzklappenprothese nach Anspruch 1 oder 2, dadurch gekennzeichnet, daß die Drehachsen in einer gemeinsamen Ebene liegen, die bei Verwendung zur Richtung der Blutströmung durch die Herzklappenprothese senkrecht ist.

4. Herzklappenprothese nach Anspruch 1, 2 oder 3, dadurch gekennzeichnet, daß das erste und zweite Blättchen (14, 16) bei etwa zwei Dritteln der Breite der Blättchen (14, 16) von den Außenkanten (36, 38) der Blättchen drehbar gehalten werden (P, P').

5. Herzklappenprothese nach Anspruch 1, 2, 3 oder 4, dadurch gekennzeichnet, daß die Krümmung der Blättchen (14, 16) kreisförmig ist.

6. Herzklappenprothese nach Anspruch 5, dadurch gekennzeichnet, daß die Krümmung C der Blättchen im Bereich von etwa 0,4 bis 0,7 liegt, wobei $C = d_0/r_0$ und $r_0$ = der Krümmungsradius der Blättchen und $d_0$ = die Länge der Sehne der Blättchen ist.

7. Herzklappenprothese nach einem der vorstehenden Ansprüche, dadurch gekennzeichnet, daß sie eine Einrichtung (30, 32) im Klappenkörper umfaßt, um den maximalen Öffnungswinkel der Blättchen auf etwa 90° von der Ebene (M–M') quer zum Durchgang zu begrenzen.

8. Herzklappenprothese nach Anspruch 7, dadurch gekennzeichnet, daß der maximale Öffnungswinkel im Bereich von etwa 85° bis 90° liegt.

**Revendications**

1. Prothèse de valvule cardiaque (10) comprenant un corps de valvule (12) ayant un passage (18) pour l'écoulement du sang au travers de l'amont vers l'aval et une première et une seconde lamelles (14, 16) portées de manière à pouvoir pivoter individuellement dans ledit passage (18) et pouvant pivoter entre une position fermée inhibant l'écoulement du sang à travers ledit passage (18) et une position ouverte permettant l'écoulement du sang, où lesdites première et seconde lamelles (14, 16) sont incurvées dans un plan normal à leur axe de rotation respectif et sont disposées de manière que les côtés convexes (26, 28) desdites première et seconde lamelles (14, 16) se font face l'un à l'autre lorsque lesdites lamelles (14, 16) sont dans ladite position ouverte, caractérisée en ce que les côtés concaves (22, 24) desdites première et seconde lamelles (14, 16) font face vers l'amont lorsqu'elles sont en position fermée et sont portées de manière à pouvoir pivoter (P, P') à plus de la moitié de la largeur des lamelles (14, 16) à partir des bords extérieurs (36, 38) desdites lamelles (14, 16).

2. Prothèse de valvule cardiaque selon la revendication 1, caractérisée en ce que lesdites première et seconde lamelles (14, 16) sont symétriques.

3. Prothèse de valvule cardiaque selon la revendication 1 ou 2, caractérisée en ce que lesdits axes de rotation se situent dans un plan commun qui est perpendiculaire à la direction d'écoulement du sang à travers ladite prothèse de valvule cardiaque utilisée.

4. Prothèse de valvule cardiaque selon la revendication 1, 2 ou 3, caractérisée en ce que lesdites première et seconde lamelles (14, 16) sont portées de manière à pouvoir pivoter (P, P') d'environ les 2/3 de la largeur des lamelles (14, 16) à partir des bords extérieurs (36, 38) desdits lamelles.

5. Prothèse de valvule cardiaque selon la revendication 1, 2, 2 ou 4, caractérisée en ce que la courbure dedites lamelles (14, 16) est circulaire.

6. Prothèse de valvule cardiaque selon la revendication 5, caractérisée en ce que la courbure (C) desdites lamelles est dans un intervalle d'environ 0,4 à 0,7 où
$C = d_0/r_0$, et
$r_0$ = rayon de courbure des lamelles, et
$d_0$ = longueur de la corde desdites lamelles.

7. Prothèse de valvule cardiaque selon l'une quelconque des revendications précédentes, caractérisée en ce qu'elles comprend des moyens (30, 32) dans ledit corps de valvule pour limiter l'angle maximum d'ouverture desdites lamelles à environ 90° à partir d'un plan (M–M') transversal par rapport audit passage.

8. Prothèse de valvule cardiaque selon la revendication 7, caractérisée en ce que l'angle maximum d'ouverture est dans un intervalle d'environ quatre-vingt-cinq à quatre-vingt-dix degrés.

FIG.1A

LEFT ATRIUM

50  52

P  Pl

M  Ml

POSTERIOR SIDE

LEFT VENTRICLE

ANTERIOR SIDE

LEFT ATRIUM

FIG.1B

14  16

P  Pl Ml

M

60  62  64

POSTERIOR SIDE

LEFT VENTRICLE

ANTERIOR SIDE

FIG.2A

FIG.2B

FIG.2C

FIG. 3

FIG.4A

FIG.4B

FIG.5

FIG.6B

FIG.6A

FIG.7B

90°

.500 .583 .667 .750

.417

C=.333

MINIMUM PEAK VELOCITY ( CM/SEC )

40.00  39.00  38.00  37.00  36.00  35.00  34.00

.65  .66  .67  .68

PIVOT POINT LOCATION

FIG.7A

85°

C=.500 .583 .667 .750

MINIMUM PEAK VELOCITY ( CM/SEC )

40.00  39.00  38.00  37.00  36.00  35.00  34.00

.65  .66  .67  .68

PIVOT POINT LOCATION

# FIG.8A

**85°**

MAXIMUM OVER PIVOT POINT LOCATION
OF MINIMUM PEAK VELOCITY ( CM/SEC )

CURVATURE

# FIG.8B

**90°**

MAXIMUM OVER PIVOT POINT LOCATION
OF MINIMUM PEAK VELOCITY (CM/SEC)

CURVATURE

EP 0 211 576 B1

FIG.9B

FIG.9A

*FIG.10A*

*FIG.10B*

*FIG. 11B*

*FIG. 11A*

.583 ** .500 .417 .333

.750

.667 ×

C = 0.000

90°

PIVOT POINT LOCATION

STROKE VOLUME / DP BAR ( CC / MM HG )

.583 .500

.750

.667

C = 0.000

85°

PIVOT POINT LOCATION

STROKE VOLUME / DP BAR ( CC / MM HG )

## FIG.12A

### TABLE I
#### PIVOT POINT THAT MAXIMIZES $V^{\star}_{MIN.}$ AT EACH CURVATURE

| MAX. OPENING ANGLE $\theta_{MAX.}$ | CURVATURE C | PIVOT POINT $Sp^{\star\star}(C)$ | MINIMUM PEAK VELOCITY $V^{\star\star}_{MIN.}(C)$ |
|---|---|---|---|
| 85° | 0. | 0.667 | 26.89 |
| | 0.500 | 0.667 | 37.00 |
| | 0.583 | 0.671 | 37.10 |
| | 0.667 | 0.674 | 37.08 |
| | 0.750 | 0.677 | 36.95 |
| 90° | 0. | 0.667 | 29.16 |
| | 0.333 | 0.660 | 37.11 |
| | 0.417 | 0.655 | 38.52 |
| | 0.500 | 0.658 | 39.05 |
| | 0.583 | 0.660 | 38.90 |
| | 0.667 | 0.661 | 38.72 |
| | 0.750 | 0.661 | 38.45 |

## FIG.12B

### TABLE II
#### PARAMETERS THAT MAXIMIZE $V^{\star}_{MIN.}$

| MAX. OPENING ANGLE $\theta_{MAX.}$ | CURVATURE $C_{OPT.}$ | PIVOT POINT $Sp^{\star\star}(C_{OPT.})$ | MINIMUM PEAK VELOCITY $V^{\star\star}_{MIN.}(C_{OPT.})$ |
|---|---|---|---|
| 85° | 0.583 | 0.671 | 37.10 |
| 90° | 0.500 | 0.658 | 39.05 |

## FIG.12C

### TABLE III
#### PARAMETERS THAT MAXIMIZE $SV/\overline{\Delta p}$

| MAX. OPENING ANGLE $\theta_{MAX.}$ | CURVATURE | PIVOT POINT | BENEFIT / COST $SV/\overline{\Delta p}$ |
|---|---|---|---|
| 85° | 0.750 | 0.667 | 7.73 |
| 90° | 0.583 | 0.667 | 7.97 |

*FIG.13*

*FIG.14*